# EUROPÄISCHE PATENTANMELDUNG

(11) **EP 3 168 303 A1**
(43) Veröffentlichungstag der Anmeldung: **17.05.2017**
(21) Anmeldenummer: 16207254.0
(22) Anmeldetag: 13.02.2006
(51) Int. Cl.: C12N 15/10

(54) **VERFAHREN ZUR ISOLIERUNG VON NUKLEINSÄUREN, WOBEI DIE NUKLEINSÄUREN BEI ERHÖHTER TEMPERATUR AN EINER MATRIX IMMOBILISIERT WERDEN**

(30) Priorität: 11.02.2005 EP 05002932
(62) Teilanmeldung aus: 06706886.6
(71) Anmelder: QIAGEN GmbH, 40724 Hilden (DE)
(72) Erfinder: SPRENGER-HAUSSELS, Markus, 40724 Hilden (DE); SCHULTE, Gabi, 40699 Erkrath (DE); DEUTSCHMANN, Thomas, 40724 Hilden (DE); FELKER, Sibylle, 40724 Hilden (DE)
(74) Vertreter: Roth, Carla

(57) **Zusammenfassung**

Die vorliegende Erfindung betrifft ein verbessertes Verfahren zur Isolierung von Nukleinsäuren wie DNA und RNA aus bakteriellen, pflanzlichen, tierischen oder humanen Zellen sowie aus Zellkulturen oder Viruskulturen.

## Beschreibung

Die vorliegende Erfindung betrifft ein verbessertes Verfahren zur Isolierung von Nukleinsäuren.

Die Isolierung von Nukleinsäuren wie DNA und RNA aus pflanzlichen, tierischen oder menschlichen Zellen sowie aus Zellkulturen oder Viruskulturen erfolgt in der Regel nach einem einheitlichen Grundmuster: die Nukleinsäuren enthaltenden Ausgangsmaterialien werden zunächst - teilweise unter Verwendung von proteinabbauenden Enzymen - aufgeschlossen. In nachfolgenden Schritten können dann einzelne Bestandteile unter Verwendung verschiedenster Methoden abgetrennt werden.

Die Abtrennung des unweigerlich in jedem Zelllysat vorkommenden Proteinanteils verkörpert dabei einen besonders wichtigen Schritt. Die Abtrennung kann beispielsweise durch Inkontaktbringen des Protein/Nukleinsäure-Gemisches mit Phenol und/oder Chloroform/Isoamylalkohol-Mischungen erfolgen. Der Proteinanteil kann aber auch durch Zusatz denaturierender Salze - wie beispielsweise Guanidiniumhydrochlorid, Guanidiniumisothiocyanant - aus der wässrigen Phase ausgefällt werden. Daneben können die Proteine durch den Zusatz von Proteasen abgebaut und anschließend entfernt werden. Abschließend kann durch den Zusatz wahlweise von DNase oder RNase die ungewünschte Nukleinsäure abgetrennt werden und die jeweils gewünschte Nukleinsäurefraktion erhalten werden. Zum Schutz der Nukleinsäuren vor einem ungewollten enzymatischen Abbau während der Isolierungsprozedur muss allerdings unter sterilen und Nuklease-freien Bedingungen gearbeitet werden. Die Trennung der Nukleinsäuren kann daneben auch auf dem Wege der Ultrazentrifugation erfolgen.

Die meisten der aus dem Stand der Technik bekannten Verfahren basieren auf einer der beiden folgenden Abtrennungsprinzipien:
Die "klassischen Verfahren" beruhen auf einem einstufigen Prozess, in dem nach Zusatz eines Puffers, der in den meisten Fällen ein Guanidiniumsalz enthält, und nach Zusatz eines organischen Extraktionsmittels - meistens Chloroform oder Phenol - eine Extraktion durchgeführt wird. Die unerwünschten Begleitstoffe werden dann mit der organischen Phase verworfen. Die in der wässrigen Phase verbleibenden Nukleinsäuren können anschließend durch eine Phasenseparation abgetrennt und isoliert werden.

Der wesentliche Nachteil dieser Verfahrensweise besteht darin, dass neben der Verwendung giftiger und gesundheitsschädigender Stoffe - wie Guanidiniumisothiocyanant, Phenol oder Chloroform - wasserlösliche Stoffe als Verunreinigung in der wässrigen Nukleinsäure-Lösung verbleiben, die in weiteren, sehr zeitaufwendigen Reinigungsschritten abgetrennt werden müssen. Diese Problematik erschwert den Einsatz dieser Verfahren zur Isolierung von Nukleinsäuren beispielsweise aus Pflanzen, da diese meist große Mengen an Polysacchariden und ähnlichen wasserlöslichen Stoffen enthalten.

Angesichts dieser Nachteile hat sich daher im Stand der Technik ein alternativer Prozess durchgesetzt, welcher auf der selektiven Adsorption von Nukleinsäuren an festen, meist mineralischen Trägern wie Siliciumdioxid, basiert. Hierbei werden in einem mehrstufigen Verfahren dem Zell- oder Viruslysat nacheinander verschiedene Pufferlösungen (Lyse-, Binde-, Wasch- und Elutionspuffer) zugesetzt; im letzten Schritt wird die gereinigte Nukleinsäure von dem Träger eluiert.

In der Fachwelt wurde zwischenzeitlich das physiko-chemische Prinzip der Bindung von Nukleinsäuren an mineralische Träger in Anwesenheit chaotroper Salze erforscht. Es wurde postuliert, dass das Binden der Nukleinsäuren an die Oberflächen der mineralischen Träger auf einer Zerstörung der übergeordneten Strukturen des wässrigen Milieus beruht, durch welche die Nukleinsäuren an die Oberfläche von mineralischen Materialien, insbesondere von Glas- bzw. Silicapartikeln, adsorbieren.

Nachteilig an den oben beschriebenen Verfahren ist insbesondere, dass beim Einsatz von Proben, die mit einem besonders hohen Anteil von störenden Sekundärstoffen angereichert sind, für die angestrebte hohe Reinheit beträchtliche Ausbeuteverluste in Kauf genommen werden müssen.

Die Aufgabe der vorliegenden Erfindung besteht somit darin, ein Verfahren zur Isolierung von RNA bzw. DNA zur Verfügung zu stellen, das die oben beschriebenen, und aus dem Stand der Technik bekannten Nachteile nicht aufweist und Nukleinsäuren in hoher Ausbeute und Reinheit verfügbar macht.

Überraschenderweise wurde gefunden, dass eine Verbesserung der Bindung von Nukleinsäure an Silicapartikel - vorzugsweise an magnetische Silicapartikel - in Gegenwart chaotroper Agenzien und/oder Alkohol, insbesondere in Gegenwart chaotroper Agenzien und Alkohol, erzielt werden kann, wenn die Nukleinsäure enthaltende Lösung vor oder während der Bindung erhitzt wird. Diese Verbesserung wirkt sich sowohl insbesondere auf die Bindung viraler RNA und DNA sowie synthetischer RNA und ebenso auf andere Nukleinsäurespezies aus.

Erfindungsgemäß werden unter biologischen Materialien Materialien auf partikulärer oder molekularer Basis verstanden. Hierzu gehören insbesondere Viren, Phagen und Zellen, wie z.B. Bakterien aber auch humane, tierische (beispielsweise Leukozyten) oder pflanzliche Zellen. Insbesondere eignet sich das erfindungsgemäße Verfahren jedoch bevorzugt für die Isolierung von Nukleinsäuren wie DNA oder RNA aus Probenmaterialien humanen oder tierischen Ursprungs - wie beispielsweise klinische Proben, wie Blut, Plasma, Serum, Mundspülflüssigkeit, Urin, Zerebralflüssigkeit, Sputum, Stuhl, Punktate, Epithelabstriche, Biopsien und andere Gewebe- oder Knochenmarkproben.

Die Probe kann auch aus dem Bereich der Umweltanalytik, der Lebensmittelanalytik oder der molekularbiologischen Forschung, z.B. aus Bakterienkulturen, Viruskulturen, Phagenlysaten, Luft- oder Wasserfilter und Produkten von Amplifikationsverfahren, z.B. wie in der PCR, stammen.

Mit dem erfindungsgemäßen Verfahren kann natives oder modifiziertes biologisches Material isoliert werden. Unter nativem biologischem Material wird Material verstanden, dessen Struktur gegenüber den natürlich vorkommenden biologischen Materialien nicht irreversibel verändert wurde. Dies schließt jedoch nicht die Modifizierung anderer Bestandteile der Probe aus. Sollen beispielsweise Zellen isoliert werden, so kann zwar das die Zellen umgebende Medium modifiziert sein, nicht jedoch die Zellen als solche. Sollen Nukleinsäuren isoliert werden, so sollen auch diese in der nativen Form, d h. nicht geschnitten oder durch Ankoppelung reaktiver Gruppen modifiziert sein. Der Begriff natives biologisches Material umfasst daher insbesondere biotinylierte Nukleinsäuren nicht. Beispiele für native biologische Materialien verkörpern u.a. virale DNA, virale RNA oder zelluläre Nukleinsäuren aus humanen oder tierischen Probenmaterialien.

Modifizierte biologische Materialien umfassen Materialien, die nicht in der Natur vorkommen, z.B. Nukleinsäuren, die durch Anheftung reaktiver, nachweisbarer, stabilisierender oder zur Immobilisierung befähigenden Gruppen modifiziert sind, z.B. biotinylierte Nukleinsäuren; daneben sind synthetische DNA und RNA aber auch beispielsweise 'armored RNA' zu nennen.

In bestimmten Fällen kann die Probe ohne Vorbehandlung in dem erfindungsgemäßen Verfahren eingesetzt werden. In vielen Fällen sollte die Probe jedoch durch eine geeignete Methode aufgeschlossen und das in der Probe enthaltende biologische Material freigesetzt werden. Verfahren zum Aufschluss von Proben sind dem Fachmann bekannt und können chemischer, enzymatischer oder physikalischer Natur sein. Auch eine Kombination dieser Verfahren ist möglich.

Hierbei können sich verschiedene Methoden für unterschiedliche biologische Materialien als vorteilhafter herausstellen, aber auf der anderen Seite ist prinzipiell jede der nachstehend aufgeführten Methoden geeignet: Lyse mit Hilfe von ionischen und nicht-ionogenen Detergenzien, wie z.B. SDS, LiDS oder Sarkosyl in geeigneten Puffern, die Verwendung von chaotropen Salzen, wie beispielsweise Guanidinhydrochlorid (GHCI), Guanidinthiocyanat (GTC), Natriumiodid, Natriumperchlorat etc.; mechanisches Auseinanderreissen, wie z.B. mittels einer French Press, Ultraschall, Mahlen mit Glaskugeln, Aluminium oder in flüssigem Stickstoff, enzymatische Lyse, beispielsweise mit Lysozym, Proteinasen, Pronasen oder Cellulasen oder einem anderen der kommerziell erhältlichen Enzyme für die Lyse; Lyse der Zelle mittels Bakteriophagen oder Virusinfektion; Gefriertrocknen; osmotischer Schock; Mikrowellenbehandlung; Temperaturbehandlung, beispielsweise Erwärmen oder Kochen oder Gefrieren, z.B. in Trockeneis oder flüssigem Stickstoff und Auftauen, alkalische Lyse.

Wie bereits oben erwähnt, stellen alle der vorstehenden Verfahren Standardtechniken zur Lyse dar, die aus dem Stand der Technik hinlänglich bekannt sind und jedes der Verfahren oder deren Kombination kann eingesetzt werden.

So ist eine Kombination aus Chaotropen und Detergenzien für die Lyse von Bakterienzellen besonders effektiv. Ein beispielhaftes, geeignetes Agens für die Lyse beinhaltet daher ein Chaotrop, wie z.B. GTC oder GHCI und ein Detergenz, wie z.B. SDS oder Sarkosyl. Diese Agentien zur Lyse können in wässriger Lösung oder in einer Pufferlösung, d. h. als so genannter Lysepuffer vorliegen. Als Puffer kann jeder geeignete Puffer, wie beispielsweise Tris, Bicin, Tricin oder Phosphatpuffer eingesetzt werden. Alternativ dazu kann das Lyseagens auch getrennt zugegeben werden. Geeignete Konzentrationen und Mengen der Lyseagentien variieren in Abhängigkeit von dem betreffenden System, Art der Zellen, etc. und können durch den Fachmann bestimmt werden, wobei beispielsweise Konzentrationen im Bereich von 2 M bis 7 M Chaotrop, wie z.B. GTC, GHCl oder Nal oder Natriumperchlorat, 0,1 M bis 1 M alkalische Agenzien, wie z.B. NaOH, und 0,1 bis 50 Gew.-% (Gewicht/Volumen) Detergenzien verwendet werden können. Ein Beispiel eines solchen Lysepuffers beinhaltet daher eine wässrige Lösung aus 4 M GTC und 1 % (Gewicht/Volumen) Sarkosyl.

Für verschiedene Lysesysteme können verschiedene Inkubationsbedingungen geeignet sein, die aus dem Stand der Technik bekannt sind. Für einen Detergenzien und/oder Chaotrope enthaltenden Lysepuffer beispielsweise kann die Inkubation bei Raumtemperatur oder bei erhöhter Temperatur, beispielsweise in einem Intervall von 37 bis 65 °C erfolgen.

Gleichermaßen kann auch die Inkubationszeit variiert werden, von wenigen Minuten bis zu 24 Stunden, beispielsweise 5 Minuten, bis zu 2 Stunden. Im Fall des GTC/Sarkosyl Lysepuffers und Bakterienzellen hat sich beispielsweise eine Inkubation bei 65°C für 10 bis 20 Minuten bewährt, die aber bei Bedarf auch variiert werden kann. Für die enzymatische Lyse, beispielsweise mittels Proteinase K etc., können längere Behandlungszeiten, beispielsweise über einen Zeitraum von 12 Stunden, erforderlich sein.

Bevorzugt erfolgt die Lyse in Gegenwart chaotroper Salze, wobei die Konzentration dieser Salze zwischen 2 und 8 mol/l beträgt, bevorzugt 4 bis 6 mol/l. Bei chaotropen Salzen handelt es sich z B. um Natriumiodid, Natriumperchlorat, Guanidininiumthiocyanat, Guanidiniumisothiocyanat oder Guanidiniumhydrochlorid. Die Bindung ist jedoch nicht auf diese Verbindungen beschränkt. Vorzugsweise findet die Bindung in Gegenwart eines Alkohols statt. Bevorzugt werden kurzkettige verzweigte oder unverzweigte Alkanole mit einem bis fünf Kohlenstoffatomen, wie z.B. Methanol, Ethanol, Propanol, Isopropanol, Butanole oder Pentanole. Dabei bewegt sich die Konzentration der Alkanole in einem Bereich von 1 bis 100% (Volumen/Volumen), ), vorzugsweise von 2 bis 80%, weiter bevorzugt von 5 bis 70 %, noch weiter bevorzugt von 10 bis 60 % und am meisten bevorzugt von 15 bis 50 %. Überraschenderweise zeigte sich, dass das Erhitzen der Nukleinsäure enthaltenden Lösung insbesondere in Gegenwart chaotroper Reagenzien und Alkohol einen ähnlichen Einfluss sowohl auf die Bindung viraler RNA und DNA als auch auf die synthetischer RNA und anderer Nukleinsäurespezies ausübt.

Zur Isolierung der Nukleinsäuren wird die Probe mit dem Trägermaterial, vorzugsweise den oben erwähnten Partikeln, in Kontakt gebracht und für eine für die Bindung ausreichende Zeit inkubiert. Für Nukleinsäuren können Inkubationszeiten zwischen 10 Sekunden und 30 Minuten zweckmäßig sein. Praktischerweise haben sich Inkubationszeiten in einem Bereich von 11 +/- 10 Minuten als vorteilhaft erwiesen.

Zur Isolieren von Nukleinsäuren werden bevorzugt silanisierte, magnetische Partikel verwendet, die perl- oder kugelförmig sind und eine Partikelgröße im Bereich von 5 bis 25 µm, vorzugsweise 6 bis 15 µm und besonders bevorzugt von 6 bis 10 µm sowie mit sehr enger Größenverteilung aufweisen. Magnetische Silicapartikel, die vorteilhafterweise in dem erfindungsgemäßen Verfahren eingesetzt werden können, sind z.B. in der internationalen Patentanmeldung WO 01/71732 Seite 6, Zeile 29 bis Seite 8, Zeile 22 beschrieben, worauf hiermit vollinhaltlich Bezug genommen wird. Erfindungsgemäß erfolgt die Bindung dabei in einem Temperaturintervall von 36 bis 75 °C, bevorzugt 46 bis 70 °C, besonders bevorzugt 50 bis 65 °C und ganz besonders bevorzugt bei 56 °C.

Ähnliche Effekte auf die Bindung von Nukleinsäuren zeigen u.a. denaturierende Substanzen wie zum Beispiel Dimethylsulfoxid.

Nach der Inkubation erfolgt die Abtrennung des biologischen Materials, vorzugsweise der Nukleinsäuren von der Probenflüssigkeit. Dies wird allgemein durch die Separation der erfindungsgemäß an die Partikel gebundenen Nukleinsäuren - bei Verwendung von magnetischen Silicapartikeln - mit Hilfe eines Magnetfeldes erreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden wurden, entfernt werden.

Diese Entfernung hängt von der Art des Gefäßes ab, in dem die Inkubation stattgefunden hat. Geeignete Verfahrensschritte zum Entfernen der Flüssigkeit sind z.B. Abpipettieren oder Absaugen der Flüssigkeit.

Falls gewünscht, können die beladenen - magnetischen - Partikel ein oder mehrmals mit einer Waschlösung gereinigt werden. Die Waschlösung wird so gewählt, dass eine Ablösung des biologischen Materials, z.B. der Nukleinsäuren, von der Partikeloberfläche möglichst nicht - bzw. nicht in einem nennenswerten Maße - stattfindet, jedoch noch vorhandene Verunreinigungen möglichst gut weggewaschen werden. Dieser Waschschritt findet bevorzugt durch Inkubation der Waschlösung mit den beladenen Partikeln statt, wobei bevorzugt eine Resuspension der Partikel vorgenommen wird, z.B. durch Schütteln oder Anlegung eines nicht mit dem ersten Magnetfeld identischen Magnetfeldes. Die verunreinigte Waschlösung wird bevorzugt genauso entfernt wie die nach der Bindung der Nukleinsäuren verbleibende Lysatflüssigkeit.

Als Waschlösung kann jeder herkömmliche Waschpuffer oder jedes andere, geeignete Medium verwendet werden. Im allgemeinen werden Puffer mit niedriger bis moderater Ionenstärke bevorzugt, wie z.B. 10 mM Tris-HCl bei einem pH von 8, 0 -10 mM NaCl. Daneben können aber auch Waschpuffer, die höhere Konzentrationen an Salzen aufweisen - wie z.B. 3 M Guanidinium-Hydrochlorid - eingesetzt werden. Ebenfalls können andere Standardmedien zur Durchführung des Waschschritts beispielsweise alkoholhaltige Medien eingesetzt werden, wie z.B. Lösungen von niederkettigen Alkanolen mit eins bis fünf Kohlenstoffatomen, bevorzugt Lösungen von Ethanol in Wasser und besonders bevorzugt wässriges 70 prozentiges Ethanol.

Die Verwendung magnetischer Partikel ermöglicht eine einfache Durchführung von Waschschritten durch die magnetische Aggregation der Partikel, Separation des Nukleinsäure-bindenden Mediums, Entfernen des Waschmediums und Zusatz von frischem Waschmedium so oft es dem Fachmann erforderlich erscheint.

Nach dem Verfahren der Nukleinsäure-Isolierung und jedem ggf. gewünschten Waschschritt kann der die Nukleinsäure tragende Träger in jedes geeignete Medium, z.B. Wasser oder einen Puffer mit niedriger Ionenstärke, transferiert, z.B. resuspendiert oder eingetaucht, werden.

Im Anschluss an den letzten Waschschritt kann ein kurzer Trocknungsschritt der magnetischen Partikel im Vakuum oder durch Ausdampfen (lassen) der Flüssigkeit vorgenommen werden.

Es ergibt sich von selbst, dass die vorstehend beschriebenen Schritte des Waschens und Trocknens nicht nur bei der Reinigung und/oder Isolierung von Nukleinsäuren durchgeführt werden können, sondern auch für die Reinigung und/oder Isolierung der anderen, vorstehend genannten biologischen Materialien geeignet sind.

In Abhängigkeit von dem Träger und der Art einer nachfolgenden Aufarbeitung kann es wünschenswert sein, die Nukleinsäure von dem Träger zu lösen oder sie nicht von dem Träger zu lösen. Im Falle eines besonderen festen Trägers, wie der oben erwähnten magnetischen Partikel, kann dieser in vielen Fällen direkt eingesetzt werden, wie z.B. in der PCR oder anderen Amplifikationsmethoden, ohne dass die Nukleinsäuren von dem Träger eluiert werden müssen. Des weiteren ist auch für viele DNA-Detektionsverfahren oder -Identifikationsverfahren ein Eluieren nicht erforderlich, da obwohl die DNA in zufälliger Weise mit der Oberfläche der Kügelchen in Berührung stehen und an einer Vielzahl von Punkten durch Wasserstoffbrückenbindungen oder ionische Bindungen oder andere Kräfte gebunden sein kann, eine hinreichende Länge von DNA für die Hybridisierung mit Oligonukleotiden und zur Vervielfältigung zur Verfügung steht.

Für den Fall, dass es sich bei dem biologischen Material um native Nukleinsäuren handelt, kann die Nukleinsäure mittels eines Elutionspuffers mit niedrigem Salzgehalt von den erfindungsgemäßen Partikeln entfernt werden. Solche Puffer sind aus dem Stand der Technik [Analytical Biochemistry 175,196-201 (1988)] bekannt. Als Elutionspuffer mit niedrigem Salzgehalt werden insbesondere Puffer mit einem Salzgehalt von weniger als 0,1 mol/l eingesetzt. Besonders bevorzugt enthält der Elutionspuffer Tris-HCl.

Besonders geeignet zur Elution ist auch entmineralisiertes Wasser.

Falls erwünscht, ist es auch möglich, die RNA von der DNA zu entfernen, was durch die Zerstörung der RNA vor dem Schritt der DNA-Separierung erreicht werden kann, beispielsweise durch Zugabe von RNase oder von Alkali, wie z.B. NaOH.

Durch Kombination der oben beschriebenen erfindungsgemäßen Isolierung von Zellen mit der ebenfalls beschriebenen erfindungsgemäßen Isolierung von Nukleinsäuren, bevorzugt durch Bindung in ihrer nativen Form an magnetische Trägermaterialien, vorzugsweise in Partikelform, bei der erfindungsgemäß erhöhten Bindetemperatur ergibt sich ein besonders vorteilhaftes Verfahren zur Isolierung von Nukleinsäuren aus zellhaltigen Proben. Die Vorteile dieser Ausführungsform besteht nicht nur in deren Einfachheit und hohen Sensitivität sowie ihrer leichten Automatisierbarkeit sondern insbesondere in einer hohen Ausbeute durch die erfindungsgemäße Bindung der Nukleinsäuren an die Silica-Oberflächen bei erhöhten Temperaturen.

Die als Folge der erfindungsgemäßen Verfahren isolierten biologischen Materialien können nun in beliebiger Weise weiter verwendet werden. Beispielsweise können sie als Substrat für verschiedene enzymatische Reaktionen verwendet werden. Im Falle der Nukleinsäuren seien als Beispiel die Sequenzierung, die radioaktive oder nichtradioaktive Markierung, die Amplifikation einer oder mehrerer in ihr enthaltender Sequenzen, die Transkription, die Hybridisierung mit markierten Sonden-Nukleinsäuren, die Translation oder die Ligation genannt. Ein Vorteil des erfindungsgemäßen Verfahrens ist, dass die Abtrennung des biologischen Materials, insbesondere der Nukleinsäuren von der Flüssigkeit, nicht nur sehr einfach ist sondern auch in hohen Ausbeuten und hohem Durchsatz durchführbar ist.

Figur 1 zeigt Mittelwerte der Ct-Werte von HCV-RNA nach real-time (RT)-PCR in Abhängigkeit von der Bindetemperatur. Eine weitere Beschreibung der Abbildung findet sich in Beispiel 1.

Figur 2 zeigt die Mittelwerte der Ct-Werte von HCV-RNA (Fig. 2 a)) HBV-DNA (Fig. 2b)) und 'armored-HIV' (Fig. 2 c)) nach real-time (RT)-PCR in Abhängigkeit von der Bindetemperatur. Eine weitere Beschreibung der Abbildung findet sich in Beispiel 2.

### Beispiel 1

Extraktion von viraler RNA und von viraler DNA (durchgeführt mit dem MagAttract Virus Mini M 48 Kit (QIAGEN, Hilden, Deutschland).

400 µl Plasma, Serum oder CSF (Liquor) werden mit Hilfe eines kommerziell erhältlichen Lysepuffers, z.B. 435 µl QIAGEN Lysis Buffer AL, enthaltend 3 µg Carrier-RNA, und einer Protease, z.B. 80 µl lyophilisierte QIAGEN Protease, resuspendiert in QIAGEN Protease Resuspension Buffer,versetzt und gemischt. Die Mischung wird über einen Zeitraum von 15 Minuten bei einer Temperatur von 56 °C inkubiert.

Danach werden die magnetischen Silica-Partikel - z.B. 30 µl MagAttract Suspension B (QIAGEN, Hilden, Deutschland) - und 525 µl Isopropanol vermischt. Es erfolgt eine 5 minütige Inkubation bei 8 °C, 18 °C, 26 °C, 36 °C, 46 °C, 56 °C, 65 °C oder 75 °C (siehe unten), während der die Nukleinsäuren an die magnetischen Silicapartikel gebunden werden. Nach dem Separieren der Partikel wird die flüssige Phase entfernt und die Partikel mit einem Waschpuffer, wie z.B. mit 500 µl QIAGEN Wash Buffer AW 1 rekonstituiert mit Ethanol, gewaschen. Nach dem Separieren der Partikel wird die flüssige Phase entfernt und die Partikel erneut gewaschen -z.B. mit 500 µl QIAGEN Wash Buffer AW 2 rekonstituiert mit Ethanol. Der letzte Waschvorgang wird wiederholt. Danach werden die Partikel mit 500 µl Ethanol gewaschen. Nach dem Separieren der Partikel wird die ethanolische Phase entfernt und die Partikel werden bei Raumtemperatur getrocknet. Anschließend wird die Nukleinsäure mit einem kommerziell erhältlichen Elutionspuffer - z.B. mit 100 µl QIAGEN AVE Elution Buffer - eluiert, die magnetischen Silicapartikel werden entfernt und das Eluat über einen Zeitraum von 5 Minuten auf eine Temperatur von 75 °C erhitzt.

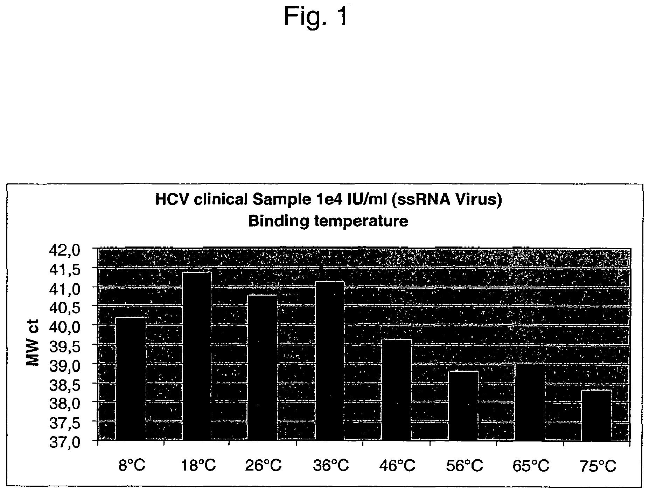

Gemäß der oben beschriebenen Methodik wurde negatives Humanplasma mit HCV (RNA-Virus) versetzt. Jeweils 12 Replikate der Proben wurden vor der Bindung auf 8 °C, 18 °C, 26 °C, 36 °C, 46 °C, 56 °C, 65 °C bzw. 75 °C temperiert. Die erhaltenen Eluate wurden jeweils einer HCV-spezifischen real-time (RT)-PCR unterzogen. Aus den in Fig. 1 dargestellten Mittelwerten der Ct-Werte in Abhängigkeit von der Bindetemperatur ist deutlich ersichtlich, dass bei der erfindungsgemäßen Verfahrensführung bei der Bindung in einem Temperaturintervall von 36 °C bis 75 °C die der Erfindung zugrunde liegende Aufgabe eindrucksvoll gelöst wird.

### Beispiel 2

In einem weiteren Experiment wurde gemäß der im oben stehenden Beispiel beschriebenen Methodik negatives Humanplasma mit HCV (einem einzelsträngigen RNA-Virus) und HBV (einem doppelsträngigen DNA-Virus) sowie 'armored-HIV' (eine synthetische RNA, die in eine Proteinhülle verpackt wurde) versetzt.

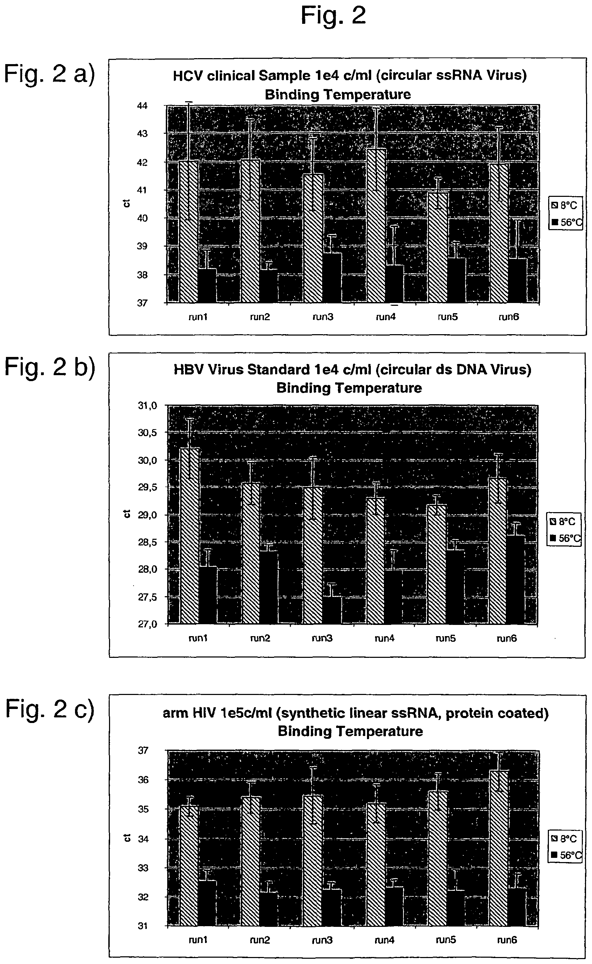

Jeweils 6 Versuchswiederholungen (runs) mit jeweils 6 Replikaten der Proben wurden zum einen mit dem Standard QIAGEN MagAttract Virus Mini M 48 Protokoll ( Protokoll wie in Beispiel 1, Bindung der Nukleinsäure jedoch bei 8°C) sowie einem modifizierten MagAttract Virus Mini M 48 Protokoll prozessiert, in welchem das Lysat vor der Bindung auf 56 °C temperiert wurde. Die erhaltenen Eluate wurden jeweils einer für HCV, HBV und HIV spezifischen real-time (RT-) PCR unterzogen. Aus den in Fig. 2 a) bis c) dargestellten Mittelwerten der Ct-Werte in Abhängigkeit von der Bindetemperatur ist deutlich ersichtlich, dass bei der erfindungsgemäßen Verfahrensführung die Bindung der Nukleinsäure bei erhöhter Temperatur die Ausbeute signifikant steigert.

## Patentansprüche

1. Verfahren zur Isolierung und/oder Reinigen von Nukleinsäuren, umfassend folgende Verfahrensschritte
a) Lysieren einer biologischen Probe,
b) Immobilisieren der freigesetzten Nukleinsäure(n) an einer Matrix auf der Basis einer oder mehrerer Silicium-Sauerstoffverbindung in Gegenwart einer chaotropen Verbindung und/oder eines verzweigten oder unverzweigten Alkanols und ggf. Waschen der auf der Matrix immobilisierten Nukleinsäure(n),
c) Abtrennen der gebundenen Nukleinsäure auf an sich bekannte Weise,
**dadurch gekennzeichnet, dass** die Immobilisierung der Nukleinsäure(n) in einem Temperaturintervall von 36 bis 75 °C erfolgt.

2. Verfahren nach Anspruch 1, **gekennzeichnet durch** eines der folgenden Merkmale
i) dass die Immobilisierung bei einer Temperatur in einem Intervall von 46 °C bis 70 °C erfolgt;
ii) dass die Immobilisierung bei einer Temperatur in einem Intervall von 50 °C bis 65 °C erfolgt;
iii) dass die Immobilisierung bei einer Temperatur von 56 °C erfolgt.

3. Verfahren nach Anspruch 1, **gekennzeichnet durch** eines der folgenden Merkmale
i) dass die Silicium-Sauerstoffverbindung **durch** Silica verkörpert wird;
ii) dass es sich bei der Matrix um magnetische Partikel mit einer Silica-Oberfläche handelt.

4. Verfahren nach Anspruch 1, **dadurch gekennzeichnet, dass** die chaotrope Verbindung durch ein chaotropes Natrium- oder Guanidiniumsalz verkörpert wird.

5. Verfahren nach Anspruch 4, **dadurch gekennzeichnet, dass** das chaotrope Natrium- oder Guanidiniumsalz Natriumiodid, Natriumperchlorat, Guanidiniumthiocyanat, Guanidinium-*iso*-thiocyanat oder Guanidiniumhydrochlorid ist oder eine Mischung zweier oder mehrerer der genannten Salze verkörpert.

6. Verfahren nach Anspruch 1, **dadurch gekennzeichnet, dass** das verzweigte oder unverzweigte Alkanol einen Alkohol mit einem bis fünf Kohlenstoffatomen verkörpert.

7. Verfahren nach Anspruch 6, **dadurch gekennzeichnet, dass** der Alkohol durch Methanol, Ethanol, *iso*-Propanpol oder durch ein verzweigtes oder unverzweigtes Butanol oder Pentanol bzw. Mischungen der genannten Alkohole verkörpert wird.

8. Verfahren nach Anspruch 6 oder 7 **dadurch gekennzeichnet, dass** das Alkanol in Form einer wässrigen Lösung in einer Konzentration von 1 bis 100 % (Volumen/Volumen), vorzugsweise von 2 bis 80%, weiter bevorzugt von 5 bis 70 %, noch weiter bevorzugt von 10 bis 60 % und am meisten bevorzugt von 15 bis 50 % vorliegt.

9. Verfahren zur Immobilisierung von Nukleinsäuren an einer Matrix auf der Basis einer Silicium-Sauerstoff-Verbindung in Gegenwart einer chaotropen Verbindung und/oder eines verzweigten oder unverzweigten Alkanols, **dadurch gekennzeichnet, dass** die Immobilisierung der Nukleinsäure(n) in einem Temperaturintervall von 36 bis 75 °C erfolgt.

10. Verfahren nach Anspruch 9, **dadurch gekennzeichnet, dass** die Immobilisierung in Gegenwart eines verzweigten oder unverzweigten Alkanols mit einem bis fünf Kohlenstoffatomen oder dessen wässriger Lösung bei einer Temperatur in einem Intervall von 46 °C bis 70 °C an einer Matrix mit einer Silicaoberfläche erfolgt.

11. Verfahren nach Anspruch 10, **dadurch gekennzeichnet, dass** die Immobilisierung in Gegenwart von Methanol, Ethanol, Propanol, iso-Propanol und/oder eines verzweigten oder unverzweigten Butanols oder Pentanols bzw. in Gegenwart einer wässrigen Lösung der genannten Alkohole oder deren Mischungen bei einer Temperatur in einem Intervall von 50 °C bis 65 °C erfolgt.

12. Verfahren nach Anspruch 11, **dadurch gekennzeichnet, dass** die Immobilisierung in Gegenwart einer wässrigen Lösung von Methanol, Ethanol, Propanol und/oder iso-Propanol in einer Konzentration in einem Intervall von 1 bis 100 % (Volumen/Volumen), vorzugsweise von 2 bis 80%, weiter bevorzugt von 5 bis 70 %, noch weiter bevorzugt von 10 bis 60 % und am meisten bevorzugt von 15 bis 50 % bei einer Temperatur von 56 °C erfolgt.

13. Verfahren nach Anspruch 9, **dadurch gekennzeichnet, dass** die chaotrope Verbindung durch ein chaotropes Natrium- oder Guanidiniumsalz verkörpert wird,
wobei optional das chaotrope Natrium- oder Guanidiniumsalz Natriumiodid, Natriumperchlorat, Guanidiniumthiocyanat, Guanidiniumisothiocyanat oder Guanidiniumhydrochlorid ist oder eine Mischung zweier oder mehrerer der genannten Salze verkörpert.

14. Verfahren nach einem der Ansprüche 9 oder 13, **gekennzeichnet durch** wenigstens eines der folgenden Merkmale
i) dass die Immobilisierung bei einer Temperatur in einem Intervall von 46 °C bis 70 °C an einer Matrix mit einer Silicaoberfläche erfolgt;
ii) dass die Immobilisierung bei einer Temperatur in einem Intervall von 50 °C bis 65 °C erfolgt;
iii) dass die Immobilisierung bei einer Temperatur 56 °C erfolgt.

15. Verfahren nach Anspruch 9, **dadurch gekennzeichnet, dass** es sich bei der Matrix um magnetische Partikel mit einer Silica-Oberfläche handelt.
